(19) Europäisches Patentamt / European Patent Office / Office européen des brevets

(11) **EP 4 210 685 B1**

(12) **EUROPEAN PATENT SPECIFICATION**

(45) Date of publication and mention of the grant of the patent:
**26.11.2025 Bulletin 2025/48**

(21) Application number: **21769152.6**

(22) Date of filing: **08.09.2021**

(51) International Patent Classification (IPC):
*A61K 31/05* (2006.01)    *A61K 45/06* (2006.01)
*A61P 25/08* (2006.01)

(52) Cooperative Patent Classification (CPC):
**A61K 31/05; A61K 45/06; A61P 25/08**

(86) International application number:
**PCT/EP2021/074661**

(87) International publication number:
**WO 2022/053486 (17.03.2022 Gazette 2022/11)**

(54) **USE OF CANNABIDIVARIN IN THE TREATMENT OF SEIZURES ASSOCIATED WITH RARE EPILEPSY SYNDROMES RELATED TO GENETIC ABNORMALITIES**

VERWENDUNG VON CANNABIDIVARIN ZUR BEHANDLUNG VON ANFÄLLEN IM ZUSAMMENHANG MIT SELTENEN EPILEPSIESYNDROMEN IN ZUSAMMENHANG MIT GENETISCHEN ANOMALIEN

UTILISATION DE CANNABIDIVARINE DANS LE TRAITEMENT DE CRISES ASSOCIÉES À DES SYNDROMES ÉPILEPTIQUES RARES LIÉS À DES ANOMALIES GÉNÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2020  GB 202014250**

(43) Date of publication of application:
**19.07.2023  Bulletin 2023/29**

(73) Proprietor: **Jazz Pharmaceuticals Research UK Limited**
**Sittingbourne, Kent ME9 8AG (GB)**

(72) Inventors:
• **CRAIG, Kevin, James**
**Sittingbourne, Kent ME9 8AG (GB)**
• **LAWSON, John, Anthony**
**Sydney, New South Wales 2052 (AU)**

(74) Representative: **HGF**
**HGF Limited**
**1 City Walk**
**Leeds LS11 9DX (GB)**

(56) References cited:
**WO-A1-2019/135075    WO-A1-2021/079148**
**GB-A- 2 487 183**

• T D M HILL ET AL: "Cannabidivarin-rich cannabis extracts are anticonvulsant in mouse and rat via a CB 1 receptor-independent mechanism", BRITISH JOURNAL OF PHARMACOLOGY, vol. 170, no. 3, 17 September 2013 (2013-09-17), UK, pages 679 - 692, XP055241333, ISSN: 0007-1188, DOI: 10.1111/bph.12321
• ANONYMOUS: "Public summary of opinion on orphan designation: Cannabidivarin for the treatment of Rett syndrome", EUROPEAN MEDICINES AGENCY, 5 January 2018 (2018-01-05), pages 1 - 4, XP055845500, Retrieved from the Internet <URL:https://www.ema.europa.eu/en/documents/orphan-designation/eu/3/17/1921-public-summary-opinion-orphan-designation-cannabidivarin-treatment-rett-syndrome_en.pdf> [retrieved on 20210928]

(Cont. next page)

EP 4 210 685 B1

- **TRISTAN T. SANDS ET AL: "Long term safety, tolerability, and efficacy of cannabidiol in children with refractory epilepsy: Results from an expanded access program in the US", CNS DRUGS, vol. 33, 1 January 2019 (2019-01-01), pages 47 - 60, XP055852436**

## Description

### FIELD OF THE INVENTION

The present invention relates to the use of cannabidivarin (CBDV) for the treatment of seizures associated with rare epilepsy syndromes. In particular the seizures associated with rare epilepsy syndromes that are treated are those which are experienced in patients diagnosed with Rett syndrome. In a further embodiment the types of seizures include focal motor seizures with impairment, focal non-motor seizures with impairment, generalised motor seizures, generalised non-motor seizures, unknown onset motor seizures, and non-motor seizures. The dose of CBDV is between 2.5 mg/kg/day to 10 mg/kg/day.

According to the present invention, the CBDV used is in the form of a highly purified extract of cannabis such that the CBDV is present at greater than 95% of the total extract (w/w) and the cannabinoid tetrahydrocannabinol (THC) has been substantially removed, to a level of not more than 1.5% (w/w).

Preferably the CBDV used is in the form of a botanically derived purified CBDV which comprises greater than or equal to 95% (w/w) CBDV and less than or equal to 5% (w/w) other cannabinoids, wherein the less than or equal to 5% (w/w) other cannabinoids comprise the cannabinoids tetrahydrocannabinol (THC); tetrahydrocannabivarin (THCV); cannabidiol-C1 (CBD-C1); cannabidiol (CBD); cannabidivarin acid (CBDVA) and cannabidiol-C4 (CBD-C4). Alternatively, a synthetically produced CBDV is used.

Where the CBDV is given concomitantly with one or more other anti-epileptic drugs (AED), the CBDV may be formulated for administration separately, sequentially or simultaneously with one or more AED or the combination may be provided in a single dosage form.

### BACKGROUND TO THE INVENTION

Epilepsy occurs in approximately 1% of the population worldwide, (Thurman *et al.,* 2011) of which 70% are able to adequately control their symptoms with the available existing anti-epileptic drugs (AED). However, 30% of this patient group, (Eadie *et al.,* 2012), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as *"failure of adequate trials of two tolerated and appropriately chosen and used AED schedules (whether as monotherapies or in combination) to achieve sustained seizure freedom"* (Kwan *et al.,* 2009).

Individuals who develop epilepsy during the first few years of life are often difficult to treat and as such are often termed treatment resistant. Children who undergo frequent seizures in childhood are often left with neurological damage which can cause cognitive, behavioral and motor delays.

Childhood epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from an investigation into the type of seizures that the patient is experiencing is undertaken. Clinical observations and electroencephalography (EEG) tests are conducted and the type(s) of seizures are classified according to the ILEA classification.

Generalized seizures, where the seizure arises within and rapidly engages bilaterally distributed networks, can be split into six subtypes: tonic-clonic (grand mal) seizures; absence (petit mal) seizures; clonic seizures; tonic seizures; atonic seizures and myoclonic seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a bilateral convulsive seizure, which is the proposed terminology to replace secondary generalized seizures (generalized seizures that have evolved from focal seizures and are no longer remain localized).

Focal seizures where the subject's awareness / responsiveness is altered are referred to as focal seizures with impairment and focal seizures where the awareness or responsiveness of the subject is not impaired are referred to as focal seizures without impairment.

Rett syndrome is a brain disorder that occurs almost exclusively in girls. After birth, girls with Rett syndrome have 6 to 18 months of apparently normal development before developing severe problems with language and communication, learning, coordination, and other brain functions.

Early in childhood, affected girls lose purposeful use of their hands and begin making repeated hand wringing, washing, or clapping motions. They tend to grow more slowly than other children and about three-quarters have a small head size (microcephaly).

Other signs and symptoms that can develop include breathing abnormalities, spitting or drooling, unusual eye movements such as intense staring or excessive blinking, cold hands and feet, irritability, sleep disturbances, seizures, and an abnormal side-to-side curvature of the spine (scoliosis).

Nearly all cases of Rett syndrome are caused by a mutation in the methyl CpG binding protein 2, or *MECP2* gene. The *MECP2* gene contains instructions for the synthesis of a protein called methyl cytosine binding protein 2 (MeCP2), which is needed for brain development and acts as one of the many biochemical switches that can either increase gene expression or tell other genes when to turn off and stop producing their own unique proteins. Because theMECP2 gene does not function properly in individuals with Rett syndrome, insufficient amounts or structurally abnormal forms of the protein are produced and can cause other genes to be abnormally expressed.

Not everyone who has an *MECP2* mutation has Rett syndrome. Mutations in the *CDKL5* and *FOXG1* genes are also associated with Rett syndrome.

Cannabidiol (CBD), a non-psychoactive derivative from the cannabis plant, has demonstrated anti-convulsant properties in several anecdotal reports, pre-clinical and clinical studies both in animal models and humans. Three randomized control trials showed efficacy of the purified pharmaceutical formulation of CBD in patients with Dravet and Lennox-Gastaut syndrome.

Based on these three trials, a botanically derived purified CBD preparation was approved by FDA in June 2018 for the treatment of seizures associated with Dravet and Lennox-Gastaut syndromes.

A trial is currently underway to test the ability of botanically derived purified CBD in the treatment of behavioural symptoms associated with Rett syndrome[1].

Cannabidivarin (CBDV) has been found to rescue the behavioural alterations in a mouse model of Rett syndrome[2]. In another study CBDV rescued memory deficits and delayed the appearance of neurological and motor defects in a mouse model of Rett syndrome[3].

GB 2487183 discloses use of CBDV in models in hippocampal brain slices, rat Pentylenetetrazole (PTZ) model of seizures, and rat Pilocarpine model of epilepsy. However, there is no mention of Rett Syndrome nor any data to show efficacy in patients with Rett Syndrome.

Amada et al. (2013)[4] further describes the use of CBDV in a PTZ model but without any mention of Rett Syndrome. Hill et al. (2013)[5] discloses a study investigating the anticonvulsant profiles of cannabis-derived botanical drug substances (BDSs). BDS used contained 47 to 58% CBDV. Again there is no mention of Rett Syndrome. An earlier paper Hill et al. in 2012[6] demonstrated that CBDV alone in fact did not have any effect on pilocarpine-induced seizures.

Huizenga et al. (2019)[7] carried out a systematic evaluation of CBDV against multiple seizure models in postnatal day (P) 10 and 20 animals. It was found that CBDV did not suppress seizures in the metl1yl-6, 7-dinlethoxy-4-ethyl-beta-carboline-3-carboxylate (DMCM) and maximal electroshock models in P10 animals.

GB 2569961 discloses a non-oil based oral pharmaceutical formulation comprising a cannabinoid, which may be a CBDV formulation. However, there is no data to show efficacy of such a formulation to treat patients with Rett Syndrome.

WO 2017/178807 relates to the use of CBDV in the treatment of autism spectrum disorder (ASD) and ASD-associated disorders such as Rett Syndrome. A MeCP2 KO mouse model of Rett Syndrome was used, which evaluates the effect on motor alterations and cognitive deficits. CBDV was found to reduce the decrease in bodyweight and survival and also improved general condition and symptoms of mobility and breathing. The impact on seizures associated with Rett Syndrome was not studied.

A public summary of opinion by the European Medicines Agency in 2018[8] mentions the use of CBDV in potentially reducing seizures in patients with Rett Syndrome. However, there is no data provided let alone any data on the specific types of seizures reduced with CBDV treatment.

The applicant has found by way of an open label, phase I trial that treatment with CBDV resulted in a significant reduction in specific seizure types including focal motor seizures with impairment, focal non-motor seizures with impairment, generalised motor seizures, generalised non-motor seizures, unknown onset motor seizures, and non-motor seizures in patients diagnosed with Rett syndrome.

## BRIEF SUMMARY OF THE DISCLOSURE

In accordance with a first aspect of the present invention there is provided a cannabidivarin (CBDV) preparation for use in the treatment of seizures associated with Rett syndrome.

In a further embodiment the seizures associated with Rett syndrome are focal motor seizures with impairment, focal non-motor seizures with impairment, generalised motor seizures, generalised non-motor seizures, unknown onset motor seizures, and non-motor seizures.

The CBDV preparation comprises greater than 95% (w/w) CBDV and not more than 1.5% (w/w) tetrahydrocannabinol (THC).

Preferably the CBDV preparation comprises greater than or equal to 95% (w/w) CBDV and less than or equal to 5% (w/w) other cannabinoids, wherein the less than or equal to 5% (w/w) other cannabinoids comprise the cannabinoids tetrahydrocannabinol (THC); tetrahydrocannabivarin (THCV); cannabidiol-C1 (CBD-C1); cannabidiol (CBD); cannabidivarin acid (CBDVA) and cannabidiol-C4 (CBD-C4).

Preferably the CBDV preparation is used in combination with one or more concomitant anti-epileptic drugs (AED).

Preferably the one or more AED is selected from the group consisting of: primidone, carbamazepine, zonegran, phenobarbitone, lamotrigine, levetiracetam, sodium valproate and clobazam.

In one embodiment the CBDV present is isolated from cannabis plant material. Preferably at least a portion of at least one of the cannabinoids present in the CBDV preparation is isolated from cannabis plant material.

In a further embodiment the CBDV is present as a synthetic preparation. Preferably at least a portion of at least one of the cannabinoids present in the CBDV preparation is prepared synthetically.

Preferably the dose of CBDV is greater than 2.5 mg/kg/day. More preferably the dose of CBDV is 10 mg/kg/day.

**DEFINITIONS**

Definitions of some of the terms used to describe the invention are detailed below:

Over 100 different cannabinoids have been identified, see for example, Handbook of Cannabis, Roger Pertwee, Chapter 1, pages 3 to 15. These cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Tonic seizures" can be generalised onset, affecting both sides of the brain, or they can be focal onset, starting in just one side of the brain. If a tonic seizure starts in both sides of the brain, all muscles tighten and the subject's body goes stiff. If standing, they may fall to the floor, their neck may extend, eyes open wide and roll upwards, whilst their arms may raise upwards and legs stretch or contract. If a tonic seizure starts in one side of the brain muscles tighten in just one area of the body. Tonic seizures usually last less than one minute.

"Clonic seizures" are characterised by sustained rhythmical jerking. During a clonic seizure, jerking of the body or parts of the body are the main symptom. They can begin in one area or affect both sides of the brain. Whilst such seizures are rare, they cannot be stopped by restraining the person.

"Tonic-clonic seizures" consist of two phases: the tonic phase and the clonic phase. In the tonic phase the body becomes entire rigid, and in the clonic phase there is uncontrolled jerking. Tonic-clonic seizures may or may not be preceded by an aura, and are often followed by headache, confusion, and sleep. They may last mere seconds or continue for several minutes. These seizures are also known as a grand mal seizure.

"Atonic seizures" occur when a person suddenly loses muscle tone so their head or body may go limp. They are also known as drop attacks. In some children, only their head drops suddenly. They can begin in one area or side of the brain (focal onset) or both sides of the brain (generalized onset).

"Myoclonic seizures" are characterised by a 'muscle jerk'. Myoclonic seizures are brief but can happen in clusters (many happening close together in time) and often happen shortly after waking. In myoclonic seizures the person is conscious, but they are classified as generalised seizures.

"Absence seizures" also may be called "petit mal" seizures. These types of seizure cause a loss of awareness for a short time. They mainly affect children although can happen at any age. During an absence seizure, a person may: stare blankly into space; look like they're "daydreaming"; flutter their eyes; make slight jerking movements of their body or limbs. The seizures usually only last up to 15 seconds and may occur several times a day.

"Focal Seizures" are defined as seizures which originate within networks limited to only one hemisphere. What happens during the seizure depends on where in the brain the seizure happens and what that part of the brain normally does.

"Focal seizures without impairment" are seizures which originate within networks limited to only one hemisphere where the awareness or responsiveness of the subject is not impaired.

"Focal seizure with impairment" usually start in a small area of the temporal lobe or frontal lobe of the brain and involve other areas of the brain within the same hemisphere that affect alertness and awareness. Most subjects experience automatisms during a focal seizure with impaired consciousness.

"Focal seizure with secondary generalisation" start in a limited area on one side of the brain and spread to involve both sides. This is different from a generalized onset seizure, which starts on both sides of the brain.

"Epileptic spasm", "spasms", "infantile spasm", "juvenile spasm" or "West syndrome" is defined as sudden flexion, extension or mixed flexion-extension of proximal and truncal muscles, lasting 1-2 seconds. Spasms typically occur in a series, usually on wakening. Subtle forms may occur with only chin movement, grimacing, or head nodding. Spasms may be bilaterally symmetric, asymmetric, or unilateral, depending on whether they are generalised onset or focal onset.

"Generalized Seizures" affect both sides of the brain or groups of cells on both sides of the brain at the same time. This term includes seizures types like tonic-clonic, absence, or atonic.

"Unknown onset seizures" occur when the beginning of the seizure is not known. A seizure could also be called an unknown onset if it is not witnessed or seen by anyone, for example when seizures happen at night or in a person who lives alone.

Seizures can also be described by whether motor symptoms occur i.e. whether movements happen during a seizure, known as a "motor seizure." When no motor symptoms happen, it can be called a "non-motor seizure."

For focal onset seizures, motor seizures may include clonic, atonic, tonic, myoclonic seizures, or epileptic spasms. There may also be automatisms or repeated automatic movements, like clapping or rubbing of hands, lipsmacking or chewing, or running. Non-motor symptoms may include changes in sensation, emotions, thinking or cognition, autonomic functions, or lack of movement (behavior arrest).

For generalized onset seizures, motor seizures usually consist of tonic-clonic seizures. Other types also include clonic, atonic, tonic, myoclonic seizures or epileptic spasms. Non-motor symptoms are usually absence seizures. Absence seizures can also have myoclonus that can affect a specific part of the body or just the eyelids.

For unknown onset seizures, motor seizures are described as either tonic-clonic or epileptic spasms. Non-motor seizures usually include a behavior arrest.

## DETAILED DESCRIPTION

### *Overview of the process*

The following describes the production of the botanically derived purified CBDV (>95% w/w) which has a known and constant composition which was used in the Example below.

Plant material harvested from the *Cannabis sativa* L. plant was subjected to liquid carbon dioxide extraction, to produce a botanical extract containing CBDV in addition to other cannabinoids and non-cannabinoid components. The extract was then further purified by a solvent crystallization method to yield botanically derived purified CBDV. The crystallization process specifically removed other cannabinoids and plant components to yield greater than 95% (w/w) CBDV.

Both the botanical starting material and the botanical extract may be controlled by specifications.

An exemplary CBDV preparation of botanically derived purified CBDV is described in Table 1.1 below. In some embodiments, the isomeric content for each cannabinoid may also be specified.

**Table 1.1: Specification of an exemplary botanically derived purified CBDV preparation**

| Test | Test Method | Limits |
|---|---|---|
| Appearance | Visual | Off-white / pale yellow crystals |
| Identification A | HPLC-UV | Retention time of major peak corresponds to certified CBDV Reference Standard |
| Identification B | GC-FID/MS | Retention time and mass spectrum of major peak corresponds to certified CBDV Reference Standard |
| Identification C | FT-IR | Conforms to reference spectrum for certified CBDV Reference Standard |
| Identification D | Melting Point | 115 - 118°C |

(continued)

| Test | Test Method | Limits |
|---|---|---|
| Identification E | Specific Optical Rotation | Conforms with certified CBDV Reference Standard; -110° to -140° (in 95% ethanol) |
| Total Purity | Calculation | $\geq$ 95.0% |
| Chromatographic Purity 1 | HPLC-UV | $\geq$ 95.0% |
| Chromatographic Purity 2 | GC-FID/MS | $\geq$ 95.0 % |
| CBD<br>CBD-C4<br>CBD-C1<br>CBDVA<br>$\Delta^9$ THC<br>THCV | HPLC-UV | NMT 4.0% w/w<br>NMT 0.2% w/w<br>NMT 0.15% w/w<br>NMT 0.15% w/w<br>NMT 0.05% w/w<br>NMT 0.01% w/w |
| Residual Solvents:<br>Alkane<br>- Ethanol | GC | NMT 0.5% w/w<br>NMT 0.5% w/w |
| Residual Water | Karl Fischer | NMT 1.0% w/w |
| i.NMT- Not more than | | |

The purity of the botanically derived purified CBDV preparation was greater than or equal to 95%. The botanically derived purified CBDV includes THC and other cannabinoids, e.g., CBD, CBDVA, THCV, CBD-C1, and CBD-C4.

Distinct chemotypes of the *Cannabis sativa* L. plant have been produced to maximize the output of the specific chemical constituents, the cannabinoids. Certain chemovars produce predominantly CBDV. Only the (-)-trans isomer of CBDV is believed to occur naturally. During purification, the stereochemistry of CBDV is not affected.

*Production of CBDV botanical drug substance*

An overview of the steps to produce a botanical extract, the intermediate, are as follows:

a. Growing
b. Direct drying
c. Decarboxylation
d. Extraction - using liquid $CO_2$
e. Winterization using ethanol
f. Filtration
g. Evaporation

High CBDV chemovars were grown, harvested, dried, baled and stored in a dry room until required. The botanical raw material (BRM) was finely chopped using an Apex mill fitted with a 1 mm screen. The milled BRM was stored in a freezer prior to extraction.

Decarboxylation of CBDVA to CBDV was carried out using heat. BRM was decarboxylated at 115°C for 60 minutes.

Extraction was performed using liquid $CO_2$ to produce botanical drug substance (BDS), which was then crystalized to produce the test material. The crude CBDV BDS was winterized to refine the extract under standard conditions (2 volumes of ethanol at -20°C for approximately 50 hours). The precipitated waxes were removed by filtration and the solvent was removed to yield the BDS.

*Production of botanically derived purified CBDV preparation*

The manufacturing steps to produce the botanically derived purified CBDV preparation from BDS were as follows:

a. Crystallization using $C_3$-$C_{12}$ straight chain or branched alkane

b. Filtration
c. Vacuum drying

The BDS produced using the methodology above was dispersed in $C_3$-$C_{12}$ straight chain or branched alkane. The mixture was manually agitated to break up any lumps and the sealed container then placed in a freezer for approximately 48 hours. The crystals were isolated via vacuum filtration, washed with aliquots of cold $C_3$-$C_{12}$ straight chain or branched alkane, and dried under a vacuum of <10mb at a temperature of 60°C until dry. The botanically derived purified CBDV preparation was stored in a freezer at -20°C in a pharmaceutical grade stainless steel container, with FDA food grade approved silicone seal and clamps.

Clearly a CBDV preparation could be produced synthetically by producing a composition with duplicate components.

Example 1 below describes the use of a botanically derived purified CBDV in an open label, phase I trial to investigate the clinical efficacy and safety of purified pharmaceutical cannabidivarin formulation (CBDV) in the treatment of patients diagnosed with Rett syndrome.

*Study design*

Subjects were required to be on one or more AEDs at stable doses for a minimum of 4 weeks prior to baseline. Subjects had to have been diagnosed with intractable epilepsy: failed adequate trial of two, or more, standard anticonvulsants, and four or more quantifiable seizures less than 8 weeks prior to screening, and two or more quantifiable seizures in prospective baseline phase.

Patients were administered botanically derived purified CBDV in a 50 mg/mL sesame oilbased solution which was titrated to an initial dose of 2.5 milligrams per kilogram per day (mg/kg/day). Doses were then increased to a goal of 10 mg/kg/day.

There were five patients in this study, and each received CBDV for various durations of time. Modifications were made to concomitant AEDs as per clinical indication.

Seizure frequency, intensity, and duration were recorded by caregivers in a diary during a baseline period of at least 28 days. Changes in seizure frequency relative to baseline were calculated after at least 2 weeks and at defined timepoints of treatment.

*Statistical Methods:*

Patients may be defined as responders if they had more than 50% reduction in seizure frequency compared to baseline. The percent change of seizure frequency for the end of the treatment period was calculated as follows:

$$\% \text{ reduction seizure frequency} = \frac{((\text{monthly seizure frequency } Baseline) - (\text{monthly seizure frequency } End))}{(\text{monthly seizure frequency } Baseline)} \times 100$$

**Results**

*Patient description*

The five patients enrolled in the open label, phase I trial were diagnosed with Rett syndrome. These patients experienced a range of different seizure types including focal seizures with impairment, generalised seizures, seizures of unknown onset, non-motor seizures and other seizure types.

The age of patients ranged from 6-13 years, all five were female as detailed in Table 1 below.

**Table 1: Patient demographics, seizure type and concomitant medication**

| Patient Number | Age (years) | Sex | Seizure types | Concomitant AEDs |
|---|---|---|---|---|
| 1 | 11 | F | Focal seizures with impairment (motor) | PRI, CAR, ZON, PHE |
| 2 | 13 | F | Focal seizures with impairment (non-motor); Other | PHE, LAM, LEV, VAL |
| 3 | 12 | F | Focal seizures with impairment (motor); Generalised tonic-clonic seizures | LEV, CLO |

(continued)

| Patient Number | Age (years) | Sex | Seizure types | Concomitant AEDs |
|---|---|---|---|---|
| 4 | F | 13 | Focal seizures with impairment (motor); Other generalized motor | VAL, CLO |
| 5 | F | 6 | Generalised tonic-clonic seizures; Other generalized (motor); Unknown onset (motor) | VAL, CLO, LEV |
| PRI = primidone, CAR = carbamazepine, ZON = zonegran, PHE = phenobarbitone, LAM = lamotrigine, LEV = levetiracetam, VAL = sodium valproate, CLO = clobazam | | | | |

*Study medication and concomitant medications*

Patients on the study were titrated up to various doses of CBDV.

Patients were taking an average of three AEDs.

*Clinical changes*

Tables 2A-2E illustrate the seizure frequency for each patient.

**Table 2A: Seizure frequency data for Patient 1**

| Onset | Movement | Baseline | /14 months |
|---|---|---|---|
| Focal seizures with impairment | Motor | 32 | 192 |
| | Non-motor | 0 | 2 |
| Generalised seizures | Motor tonic-clonic | 0 | 0 |
| | Motor other | 0 | 8 |
| Unknown onset | Motor tonic-clonic | 0 | 7 |
| | Motor other | 0 | 3 |
| Non-motor | | 0 | 0 |
| Other | | 0 | 1 |
| Total | | 32.0/month | 15.2/month |
| Mean seizure frequency reduction | | 52.5% | |

Patient 1 was treated for 14 months and experienced a 52.5% reduction in seizures over the treatment period.

**Table 2B: Seizure frequency data for Patient 2**

| Onset | Movement | Baseline | /11 months |
|---|---|---|---|
| Focal seizures with impairment | Motor | 0 | 0 |
| | Non-motor | 32 | 61 |
| Generalised seizures | Motor tonic-clonic | 0 | 0 |
| | Motor other | 0 | 0 |
| Unknown onset | Motor tonic-clonic | 0 | 0 |
| | Motor other | 0 | 0 |
| Non-motor | | 0 | 0 |
| Other | | 3 | 18 |
| Total | | 35.0/month | 7.2/month |
| Mean seizure frequency reduction | | 79.4% | |

Patient 2 was treated for 11 months and experienced a 79.4% reduction in seizures over the treatment period.

Table 2C: Seizure frequency data for Patient 3

| Onset | Movement | Baseline | /12 months |
|---|---|---|---|
| Focal seizures with impairment | Motor | 1 | 3 |
| | Non-motor | 0 | 0 |
| Generalised seizures | Motor tonic-clonic | 5 | 4 |
| | Motor other | 0 | 0 |
| Unknown onset | Motor tonic-clonic | 0 | 0 |
| | Motor other | 0 | 0 |
| Non-motor | | 0 | 0 |
| Other | | 0 | 0 |
| Total | | 6.0/month | 0.6/month |
| Mean seizure frequency reduction | | 90.0% | |

Patient 3 was treated for 12 months and experienced a 90.0% reduction in seizures over the treatment period.

Table 2D: Seizure frequency data for Patient 4

| Onset | Movement | Baseline | /12.5 months |
|---|---|---|---|
| Focal seizures with impairment | Motor | 3 | 73 |
| | Non-motor | 0 | 0 |
| Generalised seizures | Motor tonic-clonic | 0 | 0 |
| | Motor other | 4 | 4 |
| Unknown onset | Motor tonic-clonic | 0 | 0 |
| | Motor other | 0 | 0 |
| Non-motor | | 0 | 0 |
| Other | | 0 | 6 |
| Total | | 7.0/month | 6.6/month |
| Mean seizure frequency reduction | | 5.7% | |

Patient 4 was treated for 12.5 months and experienced a 5.7% reduction in seizures over the treatment period.

Table 2E: Seizure frequency data for Patient 5

| Onset | Movement | Baseline | /11 months |
|---|---|---|---|
| Focal seizures with impairment | Motor | 0 | 0 |
| | Non-motor | 0 | 0 |
| Generalised seizures | Motor tonic-clonic | 25 | 38 |
| | Motor other | 18 | 18 |
| Unknown onset | Motor tonic-clonic | 0 | 0 |
| | Motor other | 328 | 17 |
| Non-motor | | 0 | 6 |
| Other | | 0 | 0 |
| Total | | 371.0/month | 7.2/month |

(continued)

| Onset | Movement | Baseline | /11 months |
|---|---|---|---|
| **Mean seizure frequency reduction** | | 98.1% | |

Patient 5 was treated for 11 months and experienced a 98.1% reduction in seizures over the treatment period.

Overall, patients reported reductions of 5.7-98.1% in seizures over the period of treatment with CBDV.

CBDV was effective in reducing the frequency of all seizure types: focal motor seizures with impairment, focal non-motor seizures with impairment, generalised motor seizures, generalised non-motor seizures, unknown onset motor seizures, non-motor seizures and other types.

Significantly, three patients (patients 3-5) suffered from generalised seizures and all three experienced a reduction in these seizures. Four patients (patient 1-4) suffered from focal seizures with impairment and three (patients 1-3) of these patients experienced a reduction in their seizures.

Conclusions

These data indicate that CBDV was able to significantly reduce the number of seizures associated with Rett syndrome. Clearly the treatment is of significant benefit in this difficult to treat epilepsy syndrome given the high responder rate experienced in four of the five patients.

Significantly, it was found that four of the five patients had more than 50% reduction in seizure frequency compared to baseline after treatment with CBDV whilst three experienced more than 75% reduction.

In conclusion, this study signifies the use of CBDV for treatment of seizures associated with Rett syndrome. Seizure types include focal motor seizures with impairment, focal non-motor seizures with impairment, generalised motor seizures, generalised non-motor seizures, unknown onset motor seizures, and non-motor seizures for which seizure frequency rates decreased by significant rates, by up to 98%.

**References**

1. https://www.clinicaltrials.gov/ct2/show/NCT03848832?term=cannabidiol&cond=Rett+Syn drome Accessed: 10 July 2020.

2. Vigli et al. (2018) "Chronic Treatment With the Phytocannabinoid Cannabidivarin (CBDV) Rescues Behavioural Alterations and Brain Atrophy in a Mouse Model of Rett Syndrome" Neuropharmacology

3. Zamberletti et al. (2019) "Cannabidivarin completely rescues cognitive deficits and delays neurological and motor defects in male Mecp2 mutant mice" Journal of Psychopharmacology

4. Amada et al. (2013) "Cannabidivarin (CBDV) suppresses pentylenetetrazole (PTZ)-induced increases in epilepsy-related gene expression", pages 1-18, PeerJ, vol. 1.

5. Hill et al. (2013) "Cannabidivarin-rich cannabis extracts are anticonvulsant in mouse and rat via a CB1 receptor-independent mechanism", pages 679-692. British Journal of Pharmacology, vol. 170

6. Hill et al. (2012) "Cannabidivarin is anticonvulsant in mouse and rat", pages 1629-1642, British Journal of Pharmacology, vol. 167

7. Huizenga et al. (2019) "Preclinical safety and efficacy of cannabidivarin for early life seizures", pages 189-198, Neuropharmacology, vol. 148.

8. European Medicines Agency (2018) "Public summary of opinion on orphan designation: Cannabidivarin for the treatment of Rett syndrome."

## Claims

1. A cannabidivarin (CBDV) preparation for use in the treatment of seizures associated with Rett syndrome, wherein the CBDV preparation comprises greater than 95% (w/w) CBDV and not more than 1.5% (w/w) tetrahydrocannabinol (THC), and wherein the dose of CBDV is between 2.5 mg/kg/day to 10 mg/kg/day.

2. A CBDV preparation for use according to claim 1, wherein the seizures associated with Rett syndrome are focal motor seizures with impairment, focal non-motor seizures with impairment, generalised motor seizures, generalised non-motor seizures, unknown onset motor seizures, and non-motor seizures.

3. A CBDV preparation for use according to claim 1 or claim 2, wherein the CBDV preparation comprises greater than or equal to 95% (w/w) CBDV and less than or equal to 5% (w/w) other cannabinoids, wherein the less than or equal to 5% (w/w) other cannabinoids comprise the cannabinoids tetrahydrocannabinol (THC); tetrahydrocannabivarin (THCV); cannabidiol-C1 (CBD-C1); cannabidiol (CBD); cannabidivarin acid (CBDVA) and cannabidiol-C4 (CBD-C4).

4. A CBDV preparation for use according to any of the preceding claims, wherein the CBDV preparation is used in combination with one or more concomitant anti-epileptic drugs (AED).

5. A CBDV preparation for use according to claim 4, wherein the one or more AED is selected from the group consisting of: primidone, carbamazepine, zonegran, phenobarbitone, lamotrigine, levetiracetam, sodium valproate and clobazam.

6. A CBDV preparation for use according to any of the preceding claims, wherein the CBDV present is isolated from cannabis plant material.

7. A CBDV preparation for use according to any of the preceding claims, wherein at least a portion of at least one of the cannabinoids present in the CBDV preparation is isolated from cannabis plant material.

8. A CBDV preparation for use according to claims 1 to 5, wherein the CBDV is present as a synthetic preparation.

9. A CBDV preparation for use according to claim 8, wherein at least a portion of at least one of the cannabinoids present in the CBDV preparation is prepared synthetically.

10. A CBDV preparation for use according to any of the preceding claims, wherein the dose of CBDV is 10 mg/kg/day.

## Patentansprüche

1. Cannabidivarin(CBDV)-Präparat zur Verwendung bei der Behandlung von Anfällen im Zusammenhang mit dem Rett-Syndrom, wobei das CBDV-Präparat mehr als 95 Gew.-% CBDV und nicht mehr als 1,5 Gew.-% Tetrahydrocannabinol (THC) umfasst und wobei die Dosis von CBDV zwischen 2,5 mg/kg/Tag und 10 mg/kg/Tag liegt.

2. CBDV-Präparat zur Verwendung nach Anspruch 1, wobei die Anfälle im Zusammenhang mit dem Rett-Syndrom fokale motorische Anfälle mit Beeinträchtigung, fokale nichtmotorische Anfälle mit Beeinträchtigung, generalisierte motorische Anfälle, generalisierte nichtmotorische Anfälle, motorische Anfälle mit unklarem Beginn und nichtmotorische Anfälle sind.

3. CBDV-Präparat zur Verwendung nach Anspruch 1 oder Anspruch 2, wobei das CBDV-Präparat mehr als oder gleich 95 Gew.-% CBDV und weniger als oder gleich 5 Gew.-% andere Cannabinoide umfasst, wobei die weniger als oder gleich 5 Gew.-% andere Cannabinoide die Cannabinoide Tetrahydrocannabinol (THC); Tetrahydrocannabivarin (THCV); Cannabidiol-C1 (CBD-C1); Cannabidiol (CBD); Cannabidivarinsäure (CBDVA) und Cannabidiol-C4 (CBD-C4) umfassen.

4. CBDV-Präparat zur Verwendung nach einem der vorhergehenden Ansprüche, wobei das CBDV-Präparat in Kombination mit einem oder mehreren begleitenden Antiepileptika (AED) verwendet wird.

5. CBDV-Präparat zur Verwendung nach Anspruch 4, wobei das eine oder die mehreren AED ausgewählt sind aus der Gruppe, die aus Folgendem besteht: Primidon, Carbamazepin, Zonegran, Phenobarbiton, Lamotrigin, Levetiracetam, Natriumvalproat und Clobazam.

6. CBDV-Präparat zur Verwendung nach einem der vorhergehenden Ansprüche, wobei das vorliegende CBDV aus Cannabispflanzenmaterial isoliert ist.

7. CBDV-Präparat zur Verwendung nach einem der vorhergehenden Ansprüche, wobei mindestens ein Teil mindestens eines der in dem CBDV-Präparat vorliegenden Cannabinoide aus Cannabispflanzenmaterial isoliert ist.

8. CBDV-Präparat zur Verwendung nach Anspruch 1 bis 5, wobei das CBDV als ein synthetisches Präparat vorliegt.

9. CBDV-Präparat zur Verwendung nach Anspruch 8, wobei mindestens ein Teil mindestens eines der Cannabinoide, die in dem CBDV-Präparat vorliegen, synthetisch zubereitet ist.

10. CBDV-Präparat zur Verwendung nach einem der vorhergehenden Ansprüche, wobei die Dosis von CBDV 10 mg/kg/Tag beträgt.

## Revendications

1. Préparation de cannabidivarine (CBDV) destinée à être utilisée dans le traitement des crises associées au syndrome de Rett, ladite préparation de CBDV comprenant plus de 95 % (p/p) de CBDV et pas plus de 1,5 % (p/p) de tétrahydrocannabinol (THC), et ladite dose de CBDV étant comprise entre 2,5 mg/kg/jour et 10 mg/kg/jour.

2. Préparation de CBDV destiné à être utilisée selon la revendication 1, lesdites crises associées au syndrome de Rett étant des crises motrices focales avec une déficience, des crises non motrices focales avec une déficience, des crises motrices généralisées, des crises non motrices généralisées, des crises motrices à déclenchement inconnu et des crises non motrices.

3. Préparation de CBDV destiné à être utilisée selon la revendication 1 ou la revendication 2, ladite préparation de CBDV comprenant une quantité supérieure ou égale à 95 % (p/p) de CBDV et inférieure ou égale à 5 % (p/p) d'autres cannabinoïdes, lesdits autres cannabinoïdes représentant une quantité inférieure ou égale à 5 % (p/p) comprenant les cannabinoïdes tétrahydrocannabinol (THC) ; tétrahydrocannabivarine (THCV); cannabidiol-C1 (CBD-C1) ; cannabidiol (CBD) ; acide de cannabidivarine (CBDVA) et cannabidiol-C4 (CBD-C4).

4. Préparation de CBDV destiné à être utilisée selon l'une quelconque des revendications précédentes, ladite préparation de CBDV étant utilisée en combinaison avec un ou plusieurs médicaments anti-épileptiques (AED) concomitants.

5. Préparation de CBDV destiné à être utilisée selon la revendication 4, lesdits un ou plusieurs AED étant choisis dans le groupe constitué par : la primidone, la carbamazépine, le zonegran, la phénobarbitone, la lamotrigine, le lévétiracétam, le valproate de sodium et le clobazam.

6. Préparation de CBDV destiné à être utilisée selon l'une quelconque des revendications précédentes, ladite CBDV présente étant isolée à partir de matière végétale de cannabis.

7. Préparation de CBDV destiné à être utilisée selon l'une quelconque des revendications précédentes, au moins une partie d'au moins l'un des cannabinoïdes présents dans la préparation de CBDV étant isolée à partir de matière végétale de cannabis.

8. Préparation de CBDV destiné à être utilisée selon les revendications 1 à 5, ladite CBDV étant présente sous forme d'une préparation synthétique.

9. Préparation de CBDV destiné à être utilisée selon la revendication 8, au moins une partie d'au moins l'un des cannabinoïdes présents dans la préparation de CBDV étant préparée de manière synthétique.

10. Préparation de CBDV destiné à être utilisée selon l'une quelconque des revendications précédentes, ladite dose de CBDV étant de 10 mg/kg/jour.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2569961 A
- WO 2017178807 A

**Non-patent literature cited in the description**

- **ROGER PERTWEE**. Handbook of Cannabis, 3-15
- **VIGLI et al.** Chronic Treatment With the Phytocannabinoid Cannabidivarin (CBDV) Rescues Behavioural Alterations and Brain Atrophy in a Mouse Model of Rett Syndrome. *Neuropharmacology*, 2018
- **ZAMBERLETTI et al.** Cannabidivarin completely rescues cognitive deficits and delays neurological and motor defects in male Mecp2 mutant mice. *Journal of Psychopharmacology*, 2019
- **AMADA et al.** Cannabidivarin (CBDV) suppresses pentylenetetrazole (PTZ)-induced increases in epilepsy-related gene expression. *PeerJ*, 2013, vol. 1, 1-18
- **HILL et al.** Cannabidivarin-rich cannabis extracts are anticonvulsant in mouse and rat via a CB1 receptor-independent mechanism. *British Journal of Pharmacology*, 2013, vol. 170, 679-692
- **HILL et al.** Cannabidivarin is anticonvulsant in mouse and rat. *British Journal of Pharmacology*, 2012, vol. 167, 1629-1642
- **HUIZENGA et al.** Preclinical safety and efficacy of cannabidivarin for early life seizures. *Neuropharmacology*, 2019, vol. 148, 189-198
- Public summary of opinion on orphan designation: Cannabidivarin for the treatment of Rett syndrome. *European Medicines Agency*, 2018